# EUROPEAN PATENT SPECIFICATION

(11) **EP 2 100 637 B1**
(45) Date of publication and mention of the grant of the patent: **11.07.2012**
(21) Application number: 08425159.4
(22) Date of filing: 14.03.2008
(51) Int. Cl.: A61M 16/10, G01N 33/497, C12M 1/26, C12M 1/34

(54) **Respiratory apparatus with a bioburden indicator**
Atemgerät mit Gesamtkeimzahlanzeige
Appareil respiratoire doté d'un indicateur de biocharge

(43) Date of publication of application: 16.09.2009
(73) Proprietor: Covidien AG, 8212 Neuhausen am Rheinfall (CH)
(72) Inventor: Belluzzi, Camillo, 46035 Ostiglia (IT); Gallini, Sarah, 41034 Finale Emilia (IT); Solci, Massimiliano, 42015 Correggio (IT)
(74) Representative: Bergadano, Mirko

(56) References cited:
- WO-A-94/00756
- WO-A-99/02986
- WO-A-02/068580
- US-A- 5 417 204

## Description

The present invention concerns a respiratory apparatus with a bioburden indicator.

At present, respiratory devices, such as those used for mechanical ventilation in anaesthesia departments and intensive care units (ICU), do not allow the user to assess rapidly and effectively the presence of microorganisms in the respiratory device itself. Clearly, this lack exposes the patient to a possible risk of infection, the consequences of which are not always foreseeable.

For a correct assessment of the above problem, it must be borne in mind that the environments in which the respiratory devices considered are used are hospitals or similar environments, and therefore places with a high risk of infection. Moreover these devices are used on patients whose immune defence system is frequently impaired and are more endangered by any infection, caused both by pathogen and opportunist organisms.

The object of the present invention is to realise a bioburden indicator which allows the user to assess rapidly and effectively the presence of bioburden in a respiratory apparatus.

WO 94/00756 A1 discloses the features of the preambles of claims 1 and 4.

The present invention concerns a respiratory apparatus according to claim 1 and a bioburden indicator according to claim 4.

The following example is given for illustrative purposes without limitation, for a better understanding of the invention with the aid of the figures in the enclosed drawing, wherein:
- figure 1 illustrates a bioburden indicator according to the present invention and connected to a respiratory branch of a respiratory apparatus; and
- figure 2 shows an exploded view of the bioburden indicator illustrated in figure 1.

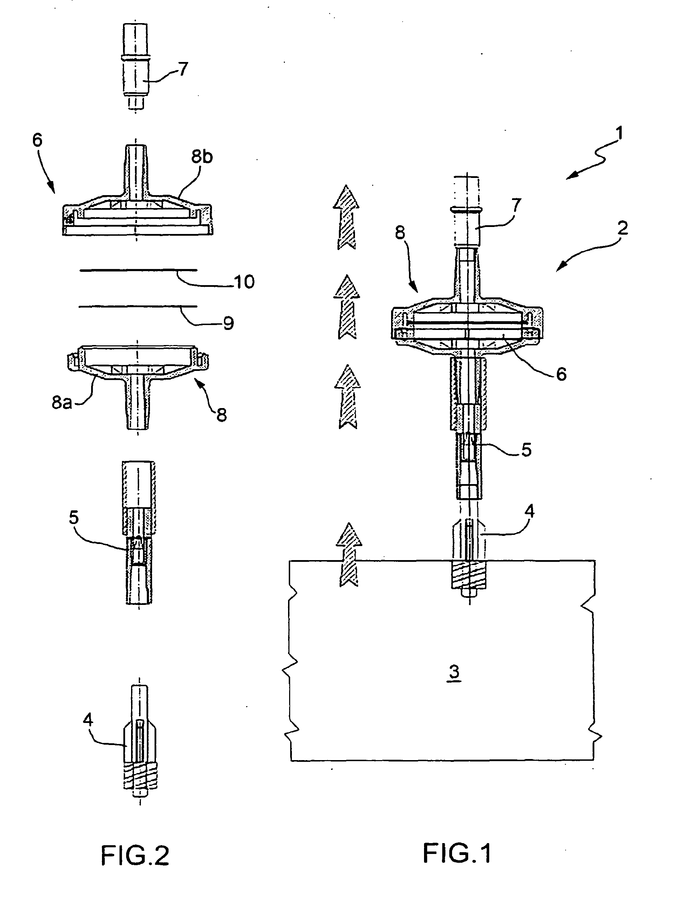

In figure 1, reference 1 indicates overall a respiratory apparatus, which is only partially illustrated for the sake of simplicity. The respiratory apparatus 1 comprises a respiratory branch 3, that could be for example a tube in a ICU ventilation system or an expiration hose of a breathing filter, a bioburden indicator 2 and a Luer connector 4 suited to connect together the bioburden indicator 2 and the respiratory branch 3.

The bioburden indicator 2 comprises, in assembly sequence, a unidirectional input valve 5 fitted on said Luer connector 4, a bioburden analysis element 6 suited to supply a visual response of the microbial burden presence, and a suction valve 7 connected to the bioburden analysis element 6 on the side opposite the input valve 5.

In use, the suction valve 7 is coupled to a suction system, for example a syringe, to suck up the air from the respiratory branch 3.

As illustrated more clearly in figure 2, the analysis element 6 comprises a containing structure 8 comprising a bottom part 8a and a top part 8b which may be connected together, an analysis disk 9 and a bacterial/viral filter 10 located between the analysis disk 9 and the suction valve 7, that is downstream from the analysis disk 9 with respect to the flow of air to be analysed.

The analysis disk 9 comprises a bioburden growth mediums able to favour the growth of any microbial burden present in the air to be analysed and to change colour when a determined level of microbial burden is reached. For this purpose, the containing structure 8 must be made of transparent material, or have at least a transparent window, so that the user can see when the analysis disk 9 changes colour.

In use, the Luer connector 4 is connected to the respiratory branch 3 by a screw connection to any sampling port, and the bioburden indicator 2 is coupled to the Luer connector 4 by a male/female coupling on the unidirectional input valve 5. Once the working conformation has been implemented as above, the suction valve 7 is connected to a suction system, by the action of which air is sucked up from the respiratory branch 3 to be brought in contact with the analysis disk 9. The unidirectional input valve 5 guarantees that the analysis element 6 is effectively isolated from the patient.

A change in colour then takes place on the analysis disk 9 depending on whether or not the microorganisms presence has exceeded a determined level of colonisation. This change will inform the user of the need to change at least the impaired parts of the respiratory device due to the high presence of bioburden.

The opportunely sealed bacterial/viral filter 10 guarantees that the bioburden present in the analysis disk 9 do not escape from the suction valve 7, with obvious advantages for the safety of the operator and of the environment.

As may appear immediate from the above description, the respiratory apparatus of the present invention offers the notable advantage of being able to know immediately the extent of bioburden presence inside respiratory branches, without this involving any type of risk for the patient or for the operator.

## Claims

1. A respiratory apparatus (1) comprising a bioburden indicator (2) that comprises an input valve (5) for the entry of air to be analysed and suited to be connected to a respiratory branch (3) by means of a connector (4), a bioburden analysis element (6), and a suction valve (7) suited to be connected to a suction system to suck up the air to be analysed from the respiratory branch (3); said respiratory apparatus being **characterised in that** said bioburden analysis element (6) is located between said input valve (5) and said suction valve (7) in such a way that the air to be analysed is forced to flow through said bioburden analysis element (6); **and in that** said bioburden analysis element (6) comprises an analysis disk (9) and a bacterial/viral filter (10) located between the analysis disk (9) and the suction valve (7); said analysis disk (9) comprising a microbial burden growth medium able to favour the growth of any microbial burden present in the air to be analysed.

2. A respiratory apparatus (1) according to claim 1, **characterised in that** the bioburden analysis element (6) comprises a colorimetric element able to change colour when a determined level of bioburden is reached.

3. A respiratory apparatus (1) according to claim 2, **characterised in that** said bioburden analysis element (6) comprises a containing structure (8) comprising at least one transparent window for viewing the change of colour.

4. A bioburden indicator (2) suited to be connected to a respiratory branch (3) of a respiratory apparatus (1) and comprising a unidirectional input valve (5) for the entry of air to be analysed coming from the respiratory branch, a bioburden analysis element (6) comprising a colorimetric element suited to change colour when a determined level of bioburden is reached, and a suction valve (7) suited to be connected to a suction system to suck up the air to be analysed from the respiratory branch (3);
said bioburden indicator (2) being **characterised in that** said bioburden analysis element (6) is located between said input valve (5) and said suction valve (7) in such a way that the air to be analysed is forced to flow through said bioburden analysis element (6); **and in that** said bioburden analysis element (6) comprises an analysis disk (9) and a bacterial/viral filter (10) located between the analysis disk (9) and the suction valve (7); said analysis disk (9) comprising a bioburden growth medium able to favour the growth of any microbial burden present in the air to be analysed.

5. A bioburden indicator (2) according to claim 4, **characterised in that** said bioburden analysis element (6) comprises a containing structure (8) comprising at least one transparent window for viewing the change of colour.

## Patentansprüche

1. Beatmungsgerät (1), das einen Biobelastungsindikator (2) umfasst, der Folgendes umfasst: ein Einströmventil (5) für den Eintritt von zu analysierender Luft, das dafür geeignet ist, über einen Verbinder (4) an eine Beatmungszweigleitung (3) angeschlossen zu werden; ein Biobelastungsanalyseelement (6); und ein Ansaugventil (7), das dafür geeignet ist, an ein Ansaugsystem angeschlossen zu werden, um die zu analysierende Luft aus der Beatmungszweigleitung (3) anzusaugen; wobei das Beatmungsgerät **dadurch gekennzeichnet ist, dass** das Biobelastungsanalyseelement (6) zwischen dem Einströmventil (5) und dem Ansaugventil (7) in einer solchen Weise angeordnet ist, dass die zu analysierende Luft veranlasst wird, durch das Biobelastungsanalyseelement (6) zu strömen; und **dadurch gekennzeichnet ist, dass** das Biobelastungsanalyseelement (6) eine Analysescheibe (9) und einen Bakterien- und Virenfilter (10), der zwischen der Analysescheibe (9) und dem Ansaugventil (7) angeordnet ist, umfasst; wobei die Analysescheibe (9) ein Mikrobenbelastungswachstumsmedium umfasst, das in der Lage ist, das Wachstum einer Mikrobenbelastung zu fördern, das in der zu analysierenden Luft vorhanden ist.

2. Beatmungsgerät (1) nach Anspruch 1, **dadurch gekennzeichnet, dass** das Biobelastungsanalyseelement (6) ein kolorimetrisches Element umfasst, das in der Lage ist, seine Farbe zu verändern, wenn ein bestimmter Biobelastungsgrad erreicht ist.

3. Beatmungsgerät (1) nach Anspruch 2, **dadurch gekennzeichnet, dass** das Biobelastungsanalyseelement (6) eine Umschließungsstruktur (8) umfasst, die mindestens ein transparentes Fenster zum Beobachten der Farbveränderung umfasst.

4. Biobelastungsindikator (2), der dafür geeignet ist, an eine Beatmungszweigleitung (3) eines Beatmungsgerätes (1) angeschlossen zu werden, und der Folgendes umfasst: ein unidirektionales Einströmventil (5) für den Eintritt von zu analysierender Luft, die von der Beatmungszweigleitung heranströmt; ein Biobelastungsanalyseelement (6), das ein kolorimetrisches Element umfasst, das dafür geeignet ist, seine Farbe zu verändern, wenn ein bestimmter Biobelastungsgrad erreicht ist; und ein Ansaugventil (7), das dafür geeignet ist, an ein Ansaugsystem angeschlossen zu werden, um die zu analysierende Luft aus der Beatmungszweigleitung (3) anzusaugen; wobei der Biobelastungsindikator (2) **dadurch gekennzeichnet ist, dass** das Biobelastungsanalyseelement (6) zwischen dem Einströmventil (5) und dem Ansaugventil (7) in einer solchen Weise angeordnet ist, dass die zu analysierende Luft veranlasst wird, durch das Biobelastungsanalyseelement (6) zu strömen; und **dadurch gekennzeichnet ist, dass** das Biobelastungsanalyseelement (6) eine Analysescheibe (9) und einen Bakterien- und Virenfilter (10), der zwischen der Analysescheibe (9) und dem Ansaugventil (7) angeordnet ist, umfasst; wobei die Analysescheibe (9) ein Mikrobenbelastungswachstumsmedium umfasst, das in der Lage ist, das Wachstum einer Mikrobenbelastung zu fördern, das in der zu analysierenden Luft vorhanden ist.

5. Biobelastungsindikator (2) nach Anspruch 4, **dadurch gekennzeichnet, dass** das Biobelastungsanalyseelement (6) eine Umschließungsstruktur (8) umfasst, die mindestens ein transparentes Fenster zum Beobachten der Farbveränderung umfasst.

## Revendications

1. Appareil respiratoire (1), comprenant un indicateur de charge biologique (2) qui comprend une vanne d'entrée (5) pour l'entrée d'air devant être analysé et appropriée pour être reliée à une branche respiratoire (3) à l'aide d'un raccord (4), un élément d'analyse de charge biologique (6), et une vanne d'aspiration (7) appropriée pour être reliée à un système d'aspiration afin d'aspirer l'air devant être analysé à partir de la branche respiratoire (3) ; ledit appareil respiratoire étant **caractérisé en ce que** ledit élément d'analyse de charge biologique (6) est disposé entre ladite vanne d'entrée (5) et ladite vanne d'aspiration (7), de telle sorte que l'air devant être analysé soit forcé à s'écouler à travers ledit élément d'analyse de charge biologique (6) ; et **en ce que** ledit élément d'analyse de charge biologique (6) comprend un disque d'analyse (9) et un filtre bactérien/viral (10) disposé entre le disque d'analyse (9) et la vanne d'aspiration (7) ; ledit disque d'analyse (9) comprenant un milieu de croissance de charge microbienne susceptible de favoriser la croissance de toute charge microbienne présente dans l'air devant être analysé.

2. Appareil respiratoire (1) selon la revendication 1, **caractérisé en ce que** l'élément d'analyse de charge biologique (6) comprend un élément colorimétrique susceptible de changer de couleur lorsqu'un niveau déterminé de charge biologique est atteint.

3. Appareil respiratoire (1) selon la revendication 2, **caractérisé en ce que** ledit élément d'analyse de charge biologique (6) comprend une structure de renfermement (8) comprenant au moins une fenêtre transparente pour visualiser le changement de couleur.

4. Indicateur de charge biologique (2) approprié pour être relié à une branche respiratoire (3) d'un appareil respiratoire (1), et comprenant une vanne d'entrée unidirectionnelle (5) pour l'entrée d'air devant être analysé à partir de la branche respiratoire, un élément d'analyse de charge biologique (6) comprenant un élément colorimétrique approprié pour changer de couleur lorsqu'un niveau prédéterminé de charge biologique est atteint, et une vanne d'aspiration (7) appropriée pour être reliée à un système d'aspiration afin d'aspirer l'air devant être analysé à partir de la branche respiratoire (3) ; ledit indicateur de charge biologique (2) étant **caractérisé en ce que** ledit élément d'analyse de charge biologique (6) est disposé entre ladite vanne d'entrée (5) et ladite vanne d'aspiration (7), de telle sorte que l'air devant être analysé soit forcé à s'écouler à travers ledit élément d'analyse de charge biologique (6) ; et **en ce que** ledit élément d'analyse de charge biologique (6) comprend un disque d'analyse (9) et un filtre bactérien/viral (10) disposé entre le disque d'analyse (9) et la vanne d'aspiration (7) ; ledit disque d'analyse (9) comprenant un milieu de croissance de charge biologique susceptible de favoriser la croissance de toute charge microbienne présente dans l'air devant être analysé.

5. Indicateur de charge biologique (2) selon la revendication 4, **caractérisé en ce que** ledit élément d'analyse de charge biologique (6) comprend une structure de renfermement (8) comprenant au moins une fenêtre transparente pour visualiser le changement de couleur.
